# EUROPÄISCHE PATENTSCHRIFT

(11) **EP 0 879 604 B1**
(45) Veröffentlichungstag und Bekanntmachung des Hinweises auf die Patenterteilung: **09.04.2003**
(21) Anmeldenummer: 97107657.5
(22) Anmeldetag: 09.05.1997
(51) Int. Cl.: A61K 47/48

(54) **Konjugat, umfassend einen Folsäureantagonisten und einen Träger**
Konjugate comprising a Folic acid antagonist and a carrier
Conjugué comprenant un antagoniste de l'acide folique et un porteur

(43) Veröffentlichungstag der Anmeldung: 25.11.1998
(73) Patentinhaber: Deutsches Krebsforschungszentrum Stiftung des öffentlichen Rechts, 69120 Heidelberg (DE)
(72) Erfinder: Sinn, Hansjörg, Dr., 69168 Wiesloch (DE); Schrenk, Hans-Herman, 67278 Zeiskam (DE); Maier-Borst, Wolfgang, Dr., 69221 Dossenheim (DE); Frei, Eva, Dr., 69124 Heidelberg (DE); Stehle, Gerd, Dr., 69123 Heidelberg (DE)
(74) Vertreter: Schüssler, Andrea, Dr.

(56) Entgegenhaltungen:
- EP-A- 0 251 455
- WO-A-85/00812
- WO-A-93/15751
- DE-A- 4 122 210
- FREI E ET AL: "Cellular uptake and metabolism of methotrexate bound to albumin differs from that of free methotrexate." EIGHTY-EIGHTH ANNUAL MEETING OF THE AMERICAN ASSOCIATION FOR CANCER RESEARCH, SAN DIEGO, CALIFORNIA, USA, APRIL 12-16, 1997. PROCEEDINGS OF THE AMERICAN ASSOCIATION FOR CANCER RESEARCH ANNUAL MEETING 38 (0). 1997. 432. ISSN: 0197-016X, XP002044230
- LEE, WILLIAM W. ET AL: "Folic acid antagonists. Methotrexate analogs containing spurious amino acids. Dichlorohomofolic acid" J. MED. CHEM. (1974), 17(3), 326-30 CODEN: JMCMAR,1974, XP002044231
- JACKMAN, A.L. ET AL: ".gamma.-linked dipeptide analogs of 2-desamino-2-methyl-N10- propargyl-5,8-dideazafolate as antitumor agents" ADV. EXP. MED. BIOL. (1993), 338(CHEMISTRY AND BIOLOGY OF PTERIDINES AND FOLATES), 579-84 CODEN: AEMBAP;ISSN: 0065-2598, Bd. 338, 1993, Seiten 579-584, XP002044232

## Beschreibung

Die Erfindung betrifft ein Konjugat, umfassend einen Folsäureantagonisten und einen Träger, Verfahren zur Herstellung eines solchen Konjugats sowie dessen Verwendung.

Folsäure ist eine in der Natur vorkommende Verbindung, die in Zellen für den Methylgruppentransfer und somit für das Wachstum von Zellen bedeutsam ist. Folsäure weist die nachstehende Formel auf:

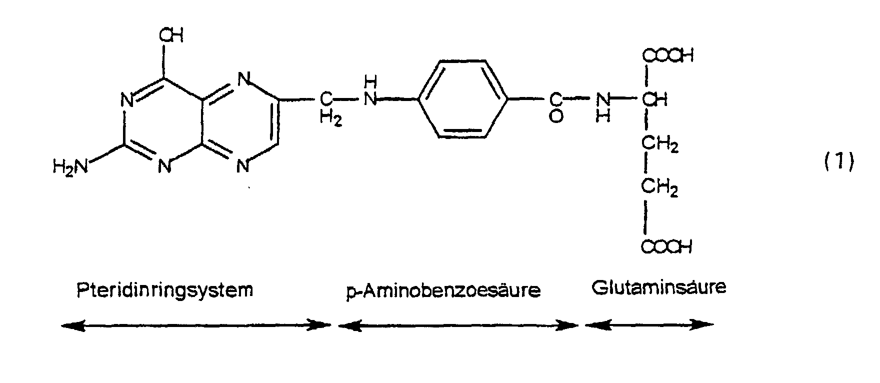

Die CH-Gruppe der Glutaminsäure stellt ein asymetrisches C-Atom dar. Folsäure existiert somit in zwei enantiomeren Formen, nämlich als D- und L-Enantiomeres.

Von diesen enantiomeren Formen findet sich aber nur das L-Enantiomere in Zellen, so daß auch nur diese Form, nicht aber das D-Enantiomere, für die Wirkung von Folsäure verantwortlich ist. Der Grund hierfür liegt darin, daß Folsäure über den Folatrezeptor in Zellen aufgenommen wird, dieser aber nur das L-Enantiomeres, nicht aber das D-Enantiomeres der Folsäure aufnimmt.

Folsäureantagonisten sind Verbindungen, die sich von Folsäure ableiten, dieser aber an ihrem Zielort, nämlich in den Zellen, entgegenwirken. Folsäureantagonisten liegen daher als L-Enantiomeres, nicht aber als D-Enantiomeres vor. Beispiele von Folsäureantagonisten sind Aminopterin und Amethopterin, das auch mit Methotrexat bezeichnet wird.

Methotrexat, d.h. das L-Enantiomere des Amethopterins, wird häufig zur Behandlung von Tumoren und Entzündungen eingesetzt. Hier haben sich allerdings große Nebenwirkungen gezeigt, da Methotrexat auch von gesundem Gewebe aufgenommen wird und für dieses toxisch ist. Lediglich die DE-A-41 22 210.5 beschreibt Konjugate aus Methotrexat und Albumin, die von Tumoren stärker aufgenommen werden als von gesundem Gewebe und somit weniger toxisch sind. Dennoch besteht ein großer Bedarf nach Mitteln, die noch geringere Nebenwirkungen aufweisen.

Lee et al., J. Med. Chem. (1974), 17(3), S. 326-330 beschreiben, daß D-MTX bei der Behandlung von Leukämie weniger aktiv ist als L-MTX.

Jackman et al., Adv. Exp. Med. Biol. (1993), 338, S. 579-584 beschreiben, daß sich die D-Enantiomere bestimmter Verbindungen zwar durch eine höhere Stabilität und eine bessere Thymidylatsynthase-Inhibition auszeichnen, aber daß dies nicht zu einer verbesserten Wachstumsinhibition von Tumorzellen führe.

EP-A-0 251 455 betrifft Antikörper-Konjugate von Aminderivaten von Folsäureanalogen.

Der vorliegenden Erfindung liegt somit die Aufgabe zugrunde, ein Mittel zur Behandlung erkrankter Gewebe, insbesondere Tumoren, bereitzustellen, das geringste Nebenwirkungen aufweist.

Erfindungsgemäß wird dies durch die Gegenstände in den Patentansprüchen erreicht.

Gegenstand der vorliegenden Erfindung ist somit ein Konjugat, das das D-Enantiomere eines Folsäureantagonisten und einen Träger umfaßt.

Die vorliegende Erfindung beruht auf der Erkenntnis des Anmelders, daß das D-Enantiomere eines Folsäureantagonisten in einem einen Träger aufweisenden Konjugat bevorzugt von erkrankten Geweben, insbesondere Tumorzellen, aufgenommen wird und in diesen eine Wirkung gegen die Erkrankung entfaltet. Ferner hat der Anmelder erkannt, daß das D-Enantiomere eines Folsäureantagonisten alleine, d.h. nicht in einem erfindungsgemäßen Konjugat vorliegend, keine Nebenwirkungen auf gesundes Gewebe hat.

Der Ausdruck "Konjugat" weist darauf hin, daß der Folsäureantagonist und der Träger kovalent verbunden sind, z.B. durch Amid- und/oder Ester-Bindungen bzw. über einen Linker.

Der Ausdruck "D-Enantiomeres eines Folsäureantagonist" umfaßt Verbindungen die sich von Folsäure ableiten, als Antagonist von Folsäure wirken und als D-Enantiomeres vorliegen. Erfindungsgemäß umfaßt das D-Enantiomere eines Folsäureantagonisten als Komponenten Pteridin, insbesondere Pterin, p-Aminobenzoesäure und eine D-Aminosäure, insbesondere D-Glutaminsäure, die gegenüber den in der Folsäure vorliegenden Komponenten chemisch verändert, d.h. modifiziert, sind. Solche Veränderungen sind z.B. Substitutionen, wie die Substitution von H-Atomen durch C₁-C₄-Alkylgruppen, insbesondere Methylgruppe, Halogenatomen, wie F, Cl, Br, I, OH- und NH₂-Gruppen, die Substitution von OH-Gruppen durch vorstehende Alkylgruppen, NH₂-Gruppen, H- und Halogenatome, sowie die Substitution von NH₂-Gruppen durch vorstehende Alkylgruppen, OH-Gruppen, H- und Halogenatome. Ferner kann eine oder können beide der Säuregruppen der Glutaminsäure als Säurederivat, z.B. als Ester oder Amid, vorliegen. Von vorstehenden Veränderungen kann eine oder können mehrere in einem erfindungsgemäß verwendeten Folsäureantagonisten vorliegen.

Vorzugsweise sind die Folsäureantagonisten D-Amethopterin ( im folgenden als D-Methotrexat bezeichnet),

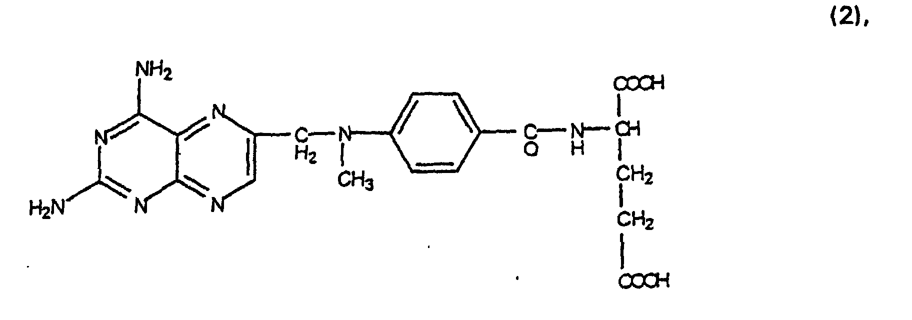

### D-Aminopterin

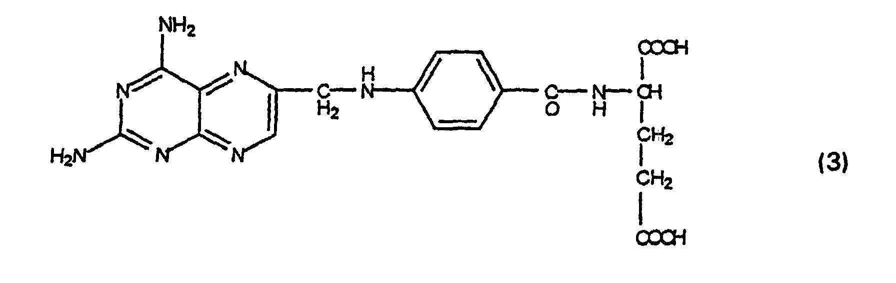

und D-e,t-FMTX (ein Methotrexat-Analog, bei dem die Glutaminsäure (Glu) durch D-erythro,threo-4-Fluoro-Glu ausgetauscht ist.

Von den Folsäureantagonisten kann einer oder können mehrere im erfindungsgemäßen Konjugat vorliegen. Liegen mehrere vor, dann können diese gleich oder verschieden voneinander sein.

Der Ausdruck "Träger" umfaßt Proteine, die nicht als körperfremd angesehen werden, und Polyether.

Die Proteine liegen vorzugsweise in nativer Form vor. In der nativen Form weisen die Proteine kein inter- und/oder intramolekulares Cross-Linking auf. Günstigerweise besitzen die Proteine ein Molekulargewicht von bis zu 100 000 Dalton, insbesondere 30 000 bis 100 000 Dalton. Ferner ist es günstig, wenn die Proteine humane Proteine sind. Beispiele der Proteine sind Albumin, Fibrinogen, Transferrin, Immunglobuline und Lipoproteine, wobei humanes Albumin bevorzugt ist. Es können auch Fragmente vorstehender Proteine verwendet werden. Ferner kann die Sequenz der Proteine bzw. der Fragmente davon Änderungen von einer oder mehreren Aminosäuren gegenüber der bekannten Sequenz der Proteine bzw. der Fragmente davon aufweisen.

Beispiele der Polyether sind Polyethylenglykole, insbesondere solche mit einem Molekulargewicht von 100 bis 20 000 Dalton. Vorzugsweise sind die Polyethylenglykole an der endständigen Hydroxylgruppe mit einer C₁-C₁₂-Alkylgruppe, insbesondere mit einer Methylgruppe, verestert oder verethert.

Ein erfindungsgemäßes Konjugat kann einen oder mehrere, insbesondere zwei, vorstehende Träger aufweisen. Liegen mehrere Träger vor, so können diese gleich oder verschieden voneinander sein. Liegen mehrere Polyether vor, so werden diese günstigerweise so gewählt, daß das Molekulargewicht aller Polyether ca. 20 000 Dalton oder mehr beträgt.

Im erfindungsgemäßen Konjugat kann der Folsäureantagonist mit dem Träger direkt kovalent oder über einen Linker verbunden sein, d.h. zwischen Träger und Folsäureantagonist liegt ein Linker vor. Als Linker sind alle Verbindungen geeignet, die Folsäureantagonist und Träger verbinden können.

Vorzugsweise ist der Linker in einer Zelle spaltbar. Der Ausdruck "Zelle" umfaßt Einzelzellen und Zellverbände. Beispiele ersterer sind körpereigene, nicht in einem Verband vorliegende Zellen. Zellverbände umfassen Gewebe, Organe und Tumoren.

Ein Linker vorstehender Art ist dem Fachmann bekannt. Auch weiß er um Faktoren, z.B. Enzyme, die in Zellen die Spaltung bestimmter chemischer Bindungen bedingen. Somit ist er in der Lage, in einer Zelle spaltbare Linker zu konstruieren. Besonders bevorzugt umfaßt ein solcher Linker eine Azo-Gruppe. Besonders günstig ist es, wenn der Linker folgende Struktur aufweist:

-Y-R-N = N-

worin
R eine organische Gruppe, vorzugsweise eine aromatische, und besonders bevorzugt Phenylen oder ein Derivat davon, ist, und
Y eine aus C(O), S(O)₂, P(O)OH und As(O)OH ausgewählte Gruppe ist.

Vorstehende Struktur eines bevorzugten Linkers entspricht jener, die der Linker in einem erfindungsgemäßen Konjugat aufweist. Ferner umfaßt die Struktur, zumidest wenn R Phenylen oder ein Derivat davon ist, eine aktive Verbindung, die sich besonders für die Therapie von Tumor-, Entzündungs- und Autoimmunerkrankungen eignet. Nach der Spaltung des Linkers und gegebenenfalls Abbau des noch am Linker gebundenen Proteins kann die Verbindung ihre volle Aktivität entfalten.

Erfindungsgemäße Konjugate können hergestellt werden, indem der Folsäureantagonist mit dem Träger und ggf. dem Linker kovalent verbunden werden. Hierzu geeignete Verfahren sowie benötigte Materialien sind dem Fachmann bekannt.

Wenn der Folsäureantagonist mindestens eine Carboxyl-Gruppe aufweist, z.B. die durch die Glutaminsäure vorliegende, können die Konjugate hergestellt werden, indem der Folsäureantagonist mit Carbodiimide und Hydroxysuccinimid zu reaktiven Succinimidylestern und diese mit dem Träger umgesetzt werden. Bei Konjugaten mit mehreren Folsäureantagonisten kann die Herstellung der Succinimidylester gemeinsam oder getrennt erfolgen.

Die Umsetzung des Folsäureantagonisten mit Carbodiimid und Hydroxysuccinimid erfolgt in einem polaren aprotischen Lösungsmittel, vorzugsweise Dimethylformamid (DMF). Das Mol-Verhältnis von Folsäureantagonist Carbodiimid : Hydroxysuccinimid beträgt etwa 1 : 1,5 : 10. Der gebildete Succinimidylester wird dann in einer wässrigen Pufferlösung, vorzugsweise NaHCO₃, mit dem Träger, wie Albumin, umgesetzt. Die Trägerkonzentration beträgt etwa 10 bis 70 mg/ml. Die so aktivierte Carboxyl-Gruppe kann dann unter Ausbildung von Säureamid- oder Säureester-Bindungen mit OH- und NH-Gruppen des Trägers reagieren, wobei erfindungsgemäße Konjugate erhalten werden. Die Konjugate können mehrfach gereinigt werden, z.B. durch Ultrafiltration, und schließlich steril filtriert werden, worauf sie applikationsfertig sind.

Erfindungsgemäße Konjugate zeichnen sich dadurch aus, daß sie über eine lange Zeit im Kreislauf des Patienten verbleiben. Ferner reichern sie sich in erkrankten Geweben, insbesondere Tumoren und Entzündungsherden an. Des weiteren zeichnen sie sich dadurch aus, daß sie noch geringere Nebenwirkungen als Konjugate mit L-Folsäureantagonisten, wie sie aus der DE-A-41 22 210.5 bekannt sind, aufweisen, wobei ihre Wirkung gegen erkrankte Gewebe, insbesondere Tumoren und Entzündungsherden erhalten bleibt.

Erfindungsgemäße Konjugate eignen sich somit bestens zur Behandlung von Tumoren, wie hämatologische und solide Tumoren, Entzündungen, z.B. Erkrankungen des rheumatischen Formenkreises, wie chronische Polyartritis, oder Psoriasis, und Autoimmunerkrankungen.

Die vorliegende Erfindung wird durch die Beispiele erläutert.

### Beispiel 1: Herstellung eines erfindungsgemäßen Konjugats aus D-Methotrexat und humanem Serumalbumin

D-Methotrexat (D-MTX) wurde in einer Konzentration von 20 mg/ml in DMF gelöst. Zu der klaren gelben Lösung wurde die 1,5 fache molare Menge an di-Cyclohexylcarbodiimid und die etwa 10 fache molare Menge an Hydroxysuccinimid gegeben. Nach einer Reaktionszeit von etwa 12 Stunden ist die Reaktion zum Succinimidylester (D-MTX-HSIE) abgeschlossen und an der auskristallisierten Menge von di-Cyclohexyl-Harnstoff (DCH) erkennbar. Die analytische Kontrolle der Reaktion wurde mittels DC durchgeführt.

| | | |
|---|---|---|
| Platten | Kieselgel 60 mit Fluoreszenzindikator, | |
| Laufmittel | Ethylacetat/MeOH : 75/25 | |
| R_{f}-Werte | D-MTX | 0,0 |
| | D-MTX-HSIE | 0,35-0,38 |

Die klare gelbe Lösung von D-MTX-HSIE in DMF wurde unter ständigem Rühren langsam zu der Proteinlösung (50-70 mg humanes Serumalbumin in 0,17 M NaHCO₃, pH 8,5) zugegeben, wobei sich nach einiger Zeit eine Trübung, bestehend aus nicht umgesetztem di-Cyclohexylcarbodiimid und noch in DMF gelöstem DCH, bildete. Nach einer Reaktionszeit von mindestens 30 Minuten wurde die Trübung über ein Sterilfilter (0,22 µm) und DMF durch Ultrafiltration mit passendem Membranfilter (YM30; Amicon) abgetrennt.

Die Reinheitskontrolle erfolgte mittels HPLC:

| | |
|---|---|
| Vorsäule | 50 x 4 mm Zorbax Diol |
| Säulen | 1. Zorbax GF 450 2. Zorbax GF 250 |
| Laufmitel | 0,2 M Phosphatpuffer pH 7,4 |
| Fluß | 1,0 ml/min |
| Druck | etwa 65 Bar |

Es wurde ein erfindungsgemäßes Konjugat aus D-Methotrexat und humanem Serumalbumin erhalten.

### Beispiel 2: Vergleich der Toxizität eines erfindungsgemäßen Konjugats aus D-Methotrexat und humanem Serumalbumin mit einem Konjugat aus Methotrexat und humanem Serumalbumin

Für diesen Versuch wurde das erfindungsgemäße Konjugat aus D-Methotrexat und humanem Serumalbumin (D-MTX-HSA) von Beispiel 1 verwendet. Ferner wurde das aus der DE-A-41 22 210.5 bekannte Konjugat aus Methotrexat und humanem Serumalbumin (MTX-HSA) eingesetzt.

Je 5 gesunden Sprague-Dawley Ratten erhielten D-MTX-HSA bzw. MTX-HSA. Es wurden jeweils 4 mg Konjugat (bezogen auf den Anteil an MTX bzw. D-MTX der Konjugate) pro kg Körpergewicht in 2 tägigem Intervall injiziert.

Die Ergebnisse sind in der Tabelle 1 angegeben.

**Tabelle 1:**

| Ergebnisse der Ermittlung der Toxizität von MTX-HSA und D-MTX- HSA nach dem 3. Tag | | | | | | |
|---|---|---|---|---|---|---|
| | Dosis/kg | SE | DU | SF | GV | Tod |
| MTX- HSA | 2 x 4 mg | 5 | 5 | 5 | 5 | 5 |
| D-MTX- HSA | 2 x 4 mg | 0 | 0 | 0 | 0 | 0 |

Abkürzungen: SE: Schleimhautentzündungen; DU: Durchfall; SF: struppiges Fell; GV: Gewichtsverlust

Wie von Tabelle 1 zu entnehmen ist, litten die Ratten bei der Verabreichung von MTX-HSA bereits ab dem 3. Tag an starken Nebenwirkungen. Am 4. Tag wurden 2 Ratten tot im Käfig aufgefunden. Die anderen 3 Ratten mußten getötet werden, da sie an sehr starken Nebenwirkungen litten. Im Gegensatz dazu wies keine der Ratten, die mit dem erfindungsgemäßen Konjugat D-MTX-HSA behandelt wurden, Nebenwirkungen auf.

Erfindungsgemäße Konjugate besitzen somit geringe Nebenwirkungen.

### Beispiel 3: Tumortherapie mit einem erfindungsgemäßen Konjugat aus D-Methotrexat und humanem Serumalbumin im Vergleich zu einem Konjugat aus Methotrexat und humanem Serumalbumin

Für diesen Versuch wurden die in Beispiel 2 angegebenen Konjugate verwendet. Als Versuchstiere wurden Walker-256 Karzinosarkom-tragende Ratten eingesetzt. Therapiebeginn war am 6. Tag nach der Tumortransplantation bei Tumorvolumina von zwischen 1000 und 2500 mm³ (Tumordurchmesser 1 x 1 bis 1 × 2 cm). Fünf Ratten erhielten MTX-HSA (3 Injektionen in 2 tägigem Abstand mit einer Dosis von 2 mg MTX-HSA (bezogen auf den MTX-Anteil) pro kg Körpergewicht). Das erfindungsgemäße Konjugat D-MTX-HSA wurde in der doppelten Dosis nach vorstehendem Protokoll verabreicht. Bei dieser Dosis traten bei MTX-HSA bereits erhebliche Nebenwirkungen auf, nicht jedoch beim erfindungegemäßen Konjugat D-MTX-HSA (vgl. Beispiel 2).

Die Ergebnisse sind in Tabelle 2 dargestellt.

**Tabelle 2:**

| Ergebnisse der Tumortherapie mit dem Konjugat MTX-HSA und dem erfindungsgemäßen Konjugat D-MTX-HSA | | | | | |
|---|---|---|---|---|---|
| | Dosis/kg | Remission | | NW | Tod |
| MTX-HSA | 3 x 2 mg | 3 | 2 | 3 | 2 |
| D-MTX-HSA | 3 x 4 mg | 5 | 0 | 0 | 0 |
| Abkürzungen: NW: Nebenwirkungen; Tod: Tod durch Tumorrezidiv | | | | | |

Die Ergebnisse in Tabelle 2 zeigen, daß in der MTX-HSA-Gruppe sich bei 3 Ratten der Tumor zurückbildete (Remission). In dieser Gruppe erlitten 2 Ratten ein Rezidiv und mußten getötet werden. Bei 3 Ratten traten Nebenwirkungen auf. Im Gegensatz dazu zeigte sich, daß bei Ratten, die mit dem erfindungsgemäßen Konjugat D-MTX-HSA behandelt wurden, alle Tiere vom Tumor geheilt wurden und dabei keine Nebenwirkungen beobachtbar waren, obwohl im Vergleich zu MTX-HSA die doppelte Dosis verwendet wurde.

Somit weisen erfindungsgemäße Konjugate geringste Nebenwirkungen auf, wobei mit diesen Konjugaten eine hervoragende Tumortherapie erzielt werden kann.

## Patentansprüche

1. Konjugat, umfassend ein D-Enantiomeres eines Folsäureantagonisten und einen Träger, **dadurch gekennzeichnet, daß** der Träger ein nicht als körperfremd angesehenes, natives Protein oder ein Polyether ist und der Folsäureantagonist ein oder mehrere Derivate von Pteridin, p-Aminobenzoesäure und einer D-Aminosäure umfaßt.

2. Konjugat nach Anspruch 1, **dadurch gekennzeichnet, daß** die D-Aminosäure Glutaminsäure ist.

3. Konjugat nach Anspruch 1, **dadurch gekennzeichnet, daß** der Folsäureantagonist D-Amethopterin oder D-Aminopterin ist.

4. Konjugat nach einem der Ansprüche 1 bis 3, **dadurch gekennzeichnet, daß** das Konjugat mehrere Folsäureantagonisten aufweist.

5. Konjugat nach Anspruch 1, **dadurch gekennzeichnet, daß** das Protein Serumalbumin ist.

6. Konjugat nach Anspruch 1, **dadurch gekennzeichnet, daß** der Polyether ein Polyethylenglykol ist.

7. Konjugat nach einem der Ansprüche 1 bis 6, **dadurch gekennzeichnet, daß** mehrere Träger vorliegen.

8. Konjugat nach einem der Ansprüche 1 bis 7, **dadurch gekennzeichnet, daß** zwischen Folsäureantagonist und Träger ein Linker vorliegt.

9. Konjugat nach Anspruch 8, **dadurch gekennzeichnet, daß** der Linker in einer Zelle spaltbar ist.

10. Konjugat nach Anspruch 9, **dadurch gekennzeichnet, daß** der Linker eine Azo-Gruppe umfaßt.

11. Verfahren zur Herstellung der Konjugate nach einem der Ansprüche 1 bis 10, **dadurch gekennzeichnet, daß** der Folsäureantagonist, der Träger und ggf. der Linker kovalent verbunden werden.

12. Verwendung der Konjugate nach einem der Ansprüche 1 bis 10 zur Herstellung einer pharmazeutischen Zusammensetzung zur Behandlung von Tumorerkrankungen, Entzündungen und/oder Autoimmunerkrankungen.

## Claims

1. A conjugate comprising a D enantiomer of a folic acid antagonist and a carrier, **characterized in that** the carrier is a native protein which is not considered foreign to the body or a polyether and the folic acid antagonist comprises one or several derivatives of pteridine, p-aminobenzoic acid and a D-amino acid.

2. The conjugate according to claim 1, **characterized in that** the D-amino acid is glutamic acid.

3. The conjugate according to claim 1, **characterized in that** the folic acid antagonist is D-amethopterine or D-aminopterine.

4. The conjugate according to any of claims 1 to 3, **characterized in that** the conjugate includes several folic acid antagonists.

5. The conjugate according to claim 1, **characterized in that** the protein is serum albumin.

6. The conjugate according to claim 1, **characterized in that** the polyether is a polyethylene glycol.

7. The conjugate according to any of claims 1 to 6, **characterized in that** several carriers are present.

8. The conjugate according to any of claims 1 to 7, **characterized in that** a linker is present between folic acid antagonist and carrier.

9. The conjugate according to claim 8, **characterized in that** the linker can be cleaved in a cell.

10. The conjugate according to claim 9, **characterized in that** the linker comprises an azo group.

11. A method of producing the conjugates according to any of claims 1 to 10, **characterized in that** the folic acid antagonist, the carrier and, where appropriate, the linker are bonded covalently.

12. Use of the conjugates according to any of claims 1 to 10 for the production of a pharmaceutical composition for treating of tumoral diseases, inflammations and/or autoimmune diseases.

## Revendications

1. Conjugué comprenant un énantiomère D d'un antagoniste de l'acide folique et un porteur, **caractérisé en ce que** le porteur est une protéine native non considérée comme étrangère à l'organisme ou un polyéther et l'antagoniste de l'acide folique comprend un ou plusieurs dérivés de ptéridine, d'acide p-aminobenzoïque et d'un D-amino-acide.

2. Conjugué suivant la revendication 1, **caractérisé en ce que** le D-amino-acide est l'acide glutamique.

3. Conjugué suivant la revendication 1, **caractérisé en ce que** l'antagoniste de l'acide folique est la D-améthoptérine ou la D-aminoptérine.

4. Conjugué suivant l'une des revendications 1 à 3, **caractérisé en ce qu'**il présente plusieurs antagonistes de l'acide folique.

5. Conjugué suivant la revendication 1, **caractérisé en ce que** la protéine est la sérum-albumine.

6. Conjugué suivant la revendication 1, **caractérisé en ce que** le polyéther est un polyéthylène-glycol.

7. Conjugué suivant l'une des revendications 1 à 6, **caractérisé en ce que** plusieurs porteurs sont présents.

8. Conjugué suivant l'une des revendications 1 à 7, **caractérisé en ce qu'**un lieur existe entre l'antagoniste de l'acide folique et le porteur.

9. Conjugué suivant la revendication 8, **caractérisé en ce que** le lieur peut être clivé dans une cellule.

10. Conjugué suivant la revendication 9, **caractérisé en ce que** le lieur comprend un groupe azo.

11. Procédé de préparation des conjugués suivant l'une des revendications 1 à 10, **caractérisé en ce que** l'antagoniste de l'acide folique, le porteur et, le cas échéant, le lieur sont liés par covalence.

12. Utilisation des conjugués suivant l'une des revendications 1 à 10 pour la préparation d'une composition pharmaceutique destinée au traitement de maladies tumorales, d'inflammations et/ou de maladies auto-immunes.
